# EUROPÄISCHE PATENTANMELDUNG

(11) **EP 2 878 333 A1**
(43) Veröffentlichungstag der Anmeldung: **03.06.2015**
(21) Anmeldenummer: 13194655.0
(22) Anmeldetag: 27.11.2013
(51) Int. Cl.: A61N 1/36, A61N 1/04

(54) **Verfahren zur Beeinflussung des visuellen Erscheinungsbildes einer Person**

(71) Anmelder: Ulrich, Frank, 40589 Düsseldorf (DE)
(72) Erfinder: Ulrich, Frank, 40589 Düsseldorf (DE)
(74) Vertreter: Stenger, Watzke & Ring

(57) **Zusammenfassung**

Die Erfindung betrifft ein Verfahren zur Beeinflussung des visuellen Erscheinungsbildes einer Person. Um eine Verbesserung des Erscheinungsbildes ohne Operationen oder medikamentöse Behandlungen zu erreichen wird mit der Erfindung eine transkranielle Stimulation des Gehirns der Person mittels eines elektrischen Stroms vorgeschlagen.

## Beschreibung

Die Erfindung betrifft ein Verfahren zur Beeinflussung des visuellen Erscheinungsbildes einer Person.

Es existieren im Stand der Technik eine Vielzahl von Verfahren, mit welchen sich das visuelle Erscheinungsbild einer Person beeinflussen lässt, insbesondere mit dem Motiv der Verbesserung. Hierzu zählen unter anderem die Verfahren der ästhetischen und plastischen Chirurgie. In diesen Bereichen werden Eingriffe sowohl aus rein ästhetischen als auch aus funktionalen Gründen durchgeführt. Zu der ästhetischen Chirurgie gehören insbesondere Eingriffe, deren Indikation nicht medizinisch, sondern ausschließlich durch den Wunsch des Patienten nach einer Verbesserung des Erscheinungsbildes bestimmt sind. Sie sind umgangssprachlich auch als sogenannte "Schönheitsoperationen" bekannt.

Die vorgenannten chirurgischen Verfahren zur Verbesserung des visuellen Erscheinungsbildes haben jedoch den Nachteil, dass diese eine Operation und/oder die Verabreichung von Medikamenten erfordern. Dadurch können Operationskomplikationen, Narkosezwischenfälle und Nebenwirkungen durch Medikamente entstehen, welche das Wohlbefinden des Patienten beeinflussen.

Auch unabhängig von chirurgischen Eingriffen werden Medikamente von Personen eingenommen, die auf eine Verbesserung ihres Aussehens hoffen. Derartige Medikamente können im einfachsten Fall allgemeine Hausmittel umfassen, bishin zu pharmazeutischen Drogen. Insgesamt stellt die Medikamenteneinnahme im kosmetischen Bereich eine erhebliche Belastung des Körpers der betroffenen Personen dar.

Darüber hinaus gibt es auch weitere Verfahren, insbesondere die Verwendung kosmetischer Cremes, Salben, Öle, Massagen, Permanent Make-ups und dergleichen. Alle bekannten Methoden stellen jedoch eine direkte Eingriffnahme in den Körper, Organe oder dergleichen dar.

Es ist daher Aufgabe der Erfindung, ein Verfahren zur Beeinflussung des visuellen Erscheinungsbildes einer Person zu schaffen, welches vollständig ohne Operationen oder Darreichung von Medikamenten auskommt.

Zur Lösung der vorgenannten Aufgabe schlägt die Erfindung ein Verfahren zur Beeinflussung des visuellen Erscheinungsbildes vor, bei welchem eine transkranielle Stimulation des Gehirns mittels eines elektrischen Stroms durchgeführt wird. Das erfindungsgemäße Verfahren lässt sich dadurch völlig schmerzfrei durchführen, wobei lediglich die leicht kribbelnde, elektrische Stimulation auf der Haut spürbar ist. Die Erfindung fußt dabei auf der Erkenntnis, dass neurologische Zustände des Gehirns auch das visuelle Erscheinungsbild der Person beeinflussen. Es ist möglich, durch die Stimulation des Gehirns mittels des elektrischen Stroms einen Zustand der Person herbeizuführen, welcher sich auch auf das visuelle Erscheinungsbild der Person auswirkt. Dabei lehnt sich die Erfindung an die wissenschaftlichen Erkenntnisse an, welche bisher im Bereich der sogenannten transkraniellen Gleichstromstimulation (transcranial directcurrent stimulation tDCS) gewonnen wurden.

In der Neurologie wird tDCS bereits erfolgreich bei depressiven Verstimmungen, Merkstörungen, motorischen Ausfallerscheinungen und zentralen Schmerzsyndromen angewendet. Desweiteren wurde eine Beeinflussung der Aufmerksamkeit und Arbeitsleistung durch transkranielle Stimulation verschiedener Hirnregionen beobachtet. Es wird zudem angenommen, dass die bei der tDCS stattfindende Veränderung der kortikalen Erregbarkeit zur Verbesserung pathologischer Veränderungen bei neurologisch oder psychiatrisch erkrankten Patienten genutzt werden kann. Auch geht man davon aus, dass in bei Depressionen pathophysiologisch relevanten Arealen sowie in verbundenen subkortikalen Regionen metabolische und biochemische Prozesse induziert werden, die eine antidepressive Wirkung haben. Hintergrund ist die physiologische Erkenntnis, dass eine anodale Stimulation von Nervenzellen, also Stimulation mit positiver Ladung, eine Depolarisation im Membranpotential des darunter liegenden Neurons verursacht, während eine kathodale Stimulation, d.h. eine Stimulation mit negativer Ladung, das negative Membranpotential hyperpolarisiert. Dabei wurde bei anodaler Stimulation eine Erhöhung der Erregbarkeit beobachtet, während bei kathodaler Stimulation eine Verminderung der Erregbarkeit eintrat. Darüber hinaus konnte bei anodaler Stimulation eine Erhöhung des zerebralen Blutflusses festgestellt werden, während bei kathodaler Stimulation der zerebrale Blutfluss abnahm. Aufgrund der Veränderung des Blutflusses ist von einer Veränderung der neuronalen Aktivität in den betroffenen Regionen auszugehen.

Nunmehr wurde im Rahmen der Erfindung überraschend festgestellt, dass die transkranielle Stimulation nicht nur geeignet ist, die vorgenannten Zustände bzw. Beschwerden auf non-invasive Art und Weise zu beeinflussen, sondern dass darüber hinaus auch das visuelle Erscheinungsbild der Person beeinflusst werden kann. Insgesamt wird somit das menschliche Nervensystem und darüber hinaus auch das visuelle Erscheinungsbild elektrokosmetisch beeinflusst, insbesondere optimiert, verbessert und verschönert. Dies wird bewirkt durch eine vorübergehende Beeinflussung, insbesondere Depolarisation oder Hyperpolarisation des zentralen Nervengewebes. Anhaltende Nebenwirkungen treten nicht auf. Allenfalls ist unter Umständen während der minutenlangen Anwendung ein leichtes Kribbeln auf der Haut zu spüren.

Es ist insbesondere vorgesehen, dass der elektrische Strom durch den Frontallappen des Gehirns geleitet wird. Vorteilhaft kommen dabei Elektroden zum Einsatz, welche den elektrischen Strom von außen zu dem Frontallappen des Gehirns leiten. Der Frontallappen füllt die vordere Schädelgrube des Kopfes. Er steuert die Ausführung von Bewegungen und reguliert kognitive Prozesse so, dass situationsgerechte Handlungen ausgeführt werden können. Die Folgen einer Schädigung des Frontallappens machen sich bemerkbar in einer ungenügenden Berücksichtigung von Handlungskonsequenzen, Haften an irrelevanten Details, mangelnde Abstimmung auf aktuelle Erfordernisse sowie auch Antriebsstörungen, Störungen der Gedächtnisleistung und Aufmerksamkeit. Da all diese Symptome gleichzeitig auch die Psyche der davon betroffenen Person beeinflussen und beispielsweise zu vermehrter Anspannung, Nervosität bis hin zu Depressionen führen können, können diese unter anderem auch einen Einfluss auf das visuelle Erscheinungsbild der Person haben. Indem somit gezielt der Frontallappen behandelt wird, kann auch eine Veränderung, insbesondere Verbesserung, des visuellen Erscheinungsbildes hervorgerufen werden.

Der elektrische Strom kann sowohl über den Stirnbereich der Person als auch über andere Kopfregionen, z. B. Hinterkopf oder Kopfseiten, zu dem Frontallappen geleitet werden. Dies ist abhängig von den Symptomen und körperlichen Parametern der jeweiligen Person, insbesondere davon, wie die Person auf eine Stimulation über Stirnbereich und/oder andere Kopfbereiche anspricht. Vorteilhaft ist die Leitung des elektrischen Gleichstroms über den Stirnbereich, da dort eine besonders kurze räumliche Distanz zu dem Frontallappen besteht.

Vorteilhaft werden die Elektroden im Stirnbereich unmittelbar über den Augenbrauen und/oder im Bereich des Haaransatzes angeordnet. Wie sich herausgestellt hat, sind dies die Bereiche über die sich das beste Behandlungsergebnis erzielen lässt.

Im Rahmen der Erfindung kann Strom impulsartig, nach einem vorgegebenen stärkevariablen Muster, mit einer vorgegebenen Frequenz oder auch als Gleichstrom eingesetzt werden. Dabei ist die Behandlungsdauer auf die zu behandelnde Person abzustimmen.

Um ein optimales Behandlungsergebnis zu erlangen, wird der elektrische Gleichstrom vorteilhaft über eine Dauer von ca. 5 bis 45 Minuten verabreicht. Die elektrische Stimulation kann dabei einmalig über einen längeren Zeitraum durchgeführt werden oder auch in zeitlich definierten Intervallen, insbesondere in Abständen von ungefähr einer Stunde zwischen aufeinanderfolgenden Stimulationen. Durch die Stimulation in zeitlich definierten Intervallen lässt sich ein gleichmäßiges und über längere Zeit verbleibendes Behandlungsergebnis erzielen.

Vorteilhaft wird der elektrische Gleichstrom mit einer Stärke von 0,5 mA bis 2 mA, insbesondere 1 mA, verabreicht. Diese Stärke kann sowohl im Rahmen einzelner Behandlungen als auch im Rahmen einer Stimulation mit zeitlich aufeinanderfolgenden Intervallen verabreicht werden. Je nach dem aktuellen neurologische Zustand der Person lässt sich die Stromstärke zu mehr oder weniger großen Beträgen korrigieren.

Es wird desweiteren vorgeschlagen, das Verfahren für die jeweilige zu behandelnde Person nach einem persönlichen Bestromungsprofil zu steuern. Das Bestromungsprofil kann eine Vielzahl von Stimulationsparametern aufweisen, wie beispielsweise Dauer und Häufigkeit der einzelnen Anwendungen, Frequenz und Stärke des Stromes, Gesamtzahl der Stimulationen während einer Anwendung, Ort der Applikation, Stromdichte, Ladungsdichte, Gesamtladung während einer Anwendung und/oder Ladung pro Stimulation. Weitere Parameter sind denkbar.

Ebenso ist neben der oben genannten Steuerung auch eine Regelung der einzelnen Stimulationsparameter möglich, wobei die während einer Stimulation angewandten Stimulationsparameter in Abhängigkeit von dem Wohlbefinden und/oder der aktuell erfolgten Beeinflussung des Erscheinungsbildes der Person variiert werden. Diese Variante ist vorteilhaft, da insbesondere der aktuelle Zustand der Person einen Einfluss darauf hat wie gut diese auf die aktuelle Stimulation anspricht. Ist die Person zum Behandlungszeitpunkt beispielsweise besonders gestresst, kann dies zu einem geringeren Ansprechen auf die Stimulation führen, so dass die Parameter angepasst werden sollten, um das gewünschte Behandlungsergebnis zu erreichen.

Dabei können auch bekannte Messverfahren, EEG und dergleichen eingesetzt werden, um wie auch immer zu ermitteln, welche Behandlungsstärke, Behandlungsdauer und welches Bestromungsprofil für die zu behandelnde Person am geeignetsten ist, um den gewünschten Effekt zu erzielen. Es lassen sich auf diese Weise individuelle Messprotokolle erstellen, die wiederum eine optimierte Behandlung ermöglichen. Die Behandlungen können stationär, in dafür vorgesehenen Einrichtungen oder auch mobil unter Verwendung entsprechender mobiler Geräte, beispielsweise Kappen und Sätze von Akkumulatoren und dergleichen, durchgeführt werden. Anwendungsdauer und Häufigkeit werden individuell festgelegt.

Neben dem vorgenannten Verfahren zur Beeinflussung des visuellen Erscheinungsbildes schlägt die Erfindung ebenfalls eine Vorrichtung zur Durchführung dieses Verfahrens vor, welche eine im Kopfbereich einer Person befestigbare Haltevorrichtung mit einer oder mehreren Elektroden aufweist. Mittels einer solchen Haltevorrichtung, welche die Elektroden bereits beinhaltet, entfällt eine separate Aufbringung jeder einzelnen Elektrode auf den Kopfbereich der Person. Dadurch kann die Vorrichtung besonders schnell und ohne Unannehmlichkeiten zeitsparend angewendet werden. Insbesondere lässt sich die Vorrichtung beispielsweise nach Art einer Mütze oder ähnlichem aufsetzen.

Es ist insbesondere vorgesehen, dass die Haltevorrichtung ein weiches, elastisches, insbesondere textiles Stirnband aufweist. Dadurch lässt sich die erfindungsgemäße Vorrichtung besonders einfach und schnell anwenden. Durch die Ausbildung als insbesondere textiles Stirnband wird eine gute Passform gewährleistet, zudem wird eine Optik geschaffen, welche nicht an eine technische Vorrichtung sondern vielmehr an ein Alltagsprodukt erinnert. Die das Verfahren anwendende Person erregt somit nicht die Aufmerksamkeit der Mitmenschen und kann das Verfahren in jedweden Alltagssituationen anwenden. Insbesondere sind die Elektroden dabei ausschließlich von der dem Kopf der Person zugewandten Seite des Stirnbandes sichtbar.

Alternativ kann ebenfalls vorgesehen sein, dass die Haltevorrichtung einen Hartkunststoff aufweist. Gemäß diesem Ausführungsbeispiel wird eine Vorrichtung geschaffen, bei welcher die Elektroden in einer unveränderlichen Position gehalten werden und im Gegensatz zu beispielsweise der Anwendung eines Stirnbandes nicht im Abstand zueinander variabel sind. Dies stellt bei nicht geübten Anwendern die Einhaltung bestimmter Elektrodenpositionen und bestimmter Elektrodenabstände sicher.

Schließlich wird mit der Erfindung auch eine Verwendung einer der vorgenannten Vorrichtungen zur Beeinflussung des visuellen Erscheinungsbildes einer Person vorgeschlagen.

Im Folgenden wird die Erfindung anhand eines Ausführungsbeispiels näher erläutert. Dabei zeigt:

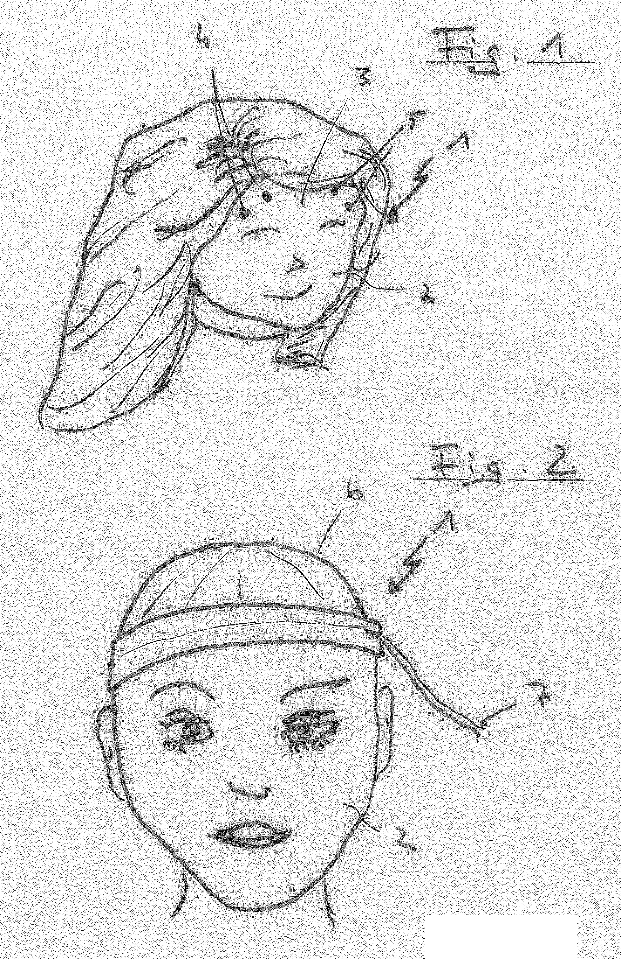

- Fig. 1:: eine schematische Darstellung beispielhafter Applikationspunkte und
- Fig. 2:: eine schematische Darstellung eines Ausführungsbeispiels für eine erfindungsgemäße Vorrichtung.

In den Figuren sind gleiche Elemente mit gleichen Bezugszeichen versehen.

Bei dem Gesicht 2 einer Person 1 kommt es im Rahmen der Erfindung auf den Stirnbereich 3 an. Dort sind üblicherweise Applikationspunkte 4 und 5 aufzufinden, welche individuell verschieden sein können. Diese müssen vorzugsweise empirisch ermittelt werden. So können sich anstelle von konkreten Punkten auch Zonen, Bereiche und dergleichen als geeignet erweisen. Sind diese bekannt, ist es möglich, die Elektroden einer stromführenden Vorrichtung in diesen Bereichen anzuordnen.

So kann beispielsweise eine Kappe 6 mit entsprechenden Leitungen und Elektroden versehen sein, die über eine Zuleitung 7 versorgt werden. Darüber hinaus können nicht gezeigte Messelektroden, Datenleitungen und dergleichen zum Einsatz kommen.

Das beschriebene Ausführungsbeispiel dient nur der Erläuterung und ist nicht beschränkend.

### Bezugszeichenliste

- 1: Person
- 2: Gesicht
- 3: Stirn
- 4: Applikationspunkt
- 5: Applikationspunkt
- 6: Kappe
- 7: Leitung

## Patentansprüche

1. Verfahren zur Beeinflussung des visuellen Erscheinungsbildes einer Person,
**gekennzeichnet durch**
eine transkranielle Stimulation des Gehirns der Person mittels eines elektrischen Stroms.

2. Verfahren nach Anspruch 1, **dadurch gekennzeichnet, dass** der elektrische Strom durch den Frontallappen des Gehirns geleitet wird.

3. Verfahren nach Anspruch 1 oder 2, **dadurch gekennzeichnet, dass** der elektrische Strom mittels Elektroden von außen über den Stirnbereich der Person zu dem Frontallappen des Gehirns geleitet wird.

4. Verfahren nach einem der vorhergehenden Ansprüche, **dadurch gekennzeichnet, dass** die Elektroden im Stirnbereich unmittelbar über den Augenbrauen und/oder im Bereich des Haaransatzes angeordnet werden.

5. Verfahren nach einem der vorhergehenden Ansprüche, **dadurch gekennzeichnet, dass** der elektrische Strom über eine Dauer von 5 bis 45 Minuten angelegt wird.

6. Verfahren nach einem der vorhergehenden Ansprüche, **dadurch gekennzeichnet, dass** der Strom impulsartig angewandt wird.

7. Verfahren nach einem der vorhergehenden Ansprüche, **dadurch gekennzeichnet, dass** Gleichstrom eingesetzt wird.

8. Verfahren nach einem der vorhergehenden Ansprüche, **dadurch gekennzeichnet, dass** elektrischer Gleichstrom mit einer Stärke von 0,5 mA bis 2 mA verabreicht wird.

9. Verfahren nach einem der vorhergehenden Ansprüche, **dadurch gekennzeichnet, dass** die Stimulation in zeitlich definierten Intervallen, insbesondere in Abständen von 1 Stunde zwischen aufeinanderfolgenden Stimulationen, durchgeführt wird.

10. Verfahren nach einem der vorhergehenden Ansprüche, **dadurch gekennzeichnet dass** die Stimulation nach einem für die zu behandelnde Person charakteristischen Bestromungsprofil ausgeführt wird.

11. Vorrichtung zur Durchführung des Verfahrens nach einem der vorhergehenden Ansprüche,
**gekennzeichnet durch**
eine im Kopfbereich einer Person befestigbare Haltevorrichtung, welche eine oder mehrere Elektroden aufweist.

12. Vorrichtung nach Anspruch 11, **dadurch gekennzeichnet, dass** die Haltevorrichtung ein weiches, elastisches, insbesondere textiles Stirnband aufweist.

13. Vorrichtung nach Anspruch 12, **dadurch gekennzeichnet, dass** die Elektroden ausschließlich von der dem Kopf der Person zugewandten Seite des Stirnbandes sichtbar sind.

14. Vorrichtung nach Anspruch 11, **dadurch gekennzeichnet, dass** die Haltevorrichtung einen Hartkunststoff aufweist.

15. Verwendung einer Vorrichtung gemäß einem der Ansprüche 11 bis 14 zur Beeinflussung des visuellen Erscheinungsbildes einer Person.

## Geänderte Patentansprüche

### Geänderte Patentansprüche gemäss Regel 137(2) EPÜ.

1. Elektrokosmetisches Verfahren zur Beeinflussung des visuellen Erscheinungsbildes einer Person,
**gekennzeichnet durch**
eine transkranielle Stimulation des Gehirns der Person mittels eines elektrischen Stroms.

2. Verfahren nach Anspruch 1, **dadurch gekennzeichnet, dass** der elektrische Strom durch den Frontallappen des Gehirns geleitet wird.

3. Verfahren nach Anspruch 1 oder 2, **dadurch gekennzeichnet, dass** der elektrische Strom mittels Elektroden von außen über den Stirnbereich der Person zu dem Frontallappen des Gehirns geleitet wird.

4. Verfahren nach einem der vorhergehenden Ansprüche, **dadurch gekennzeichnet, dass** die Elektroden im Stirnbereich unmittelbar über den Augenbrauen und/oder im Bereich des Haaransatzes angeordnet werden.

5. Verfahren nach einem der vorhergehenden Ansprüche, **dadurch gekennzeichnet, dass** der elektrische Strom über eine Dauer von 5 bis 45 Minuten angelegt wird.

6. Verfahren nach einem der vorhergehenden Ansprüche, **dadurch gekennzeichnet, dass** der Strom impulsartig angewandt wird.

7. Verfahren nach einem der vorhergehenden Ansprüche, **dadurch gekennzeichnet, dass** Gleichstrom eingesetzt wird.

8. Verfahren nach einem der vorhergehenden Ansprüche, **dadurch gekennzeichnet, dass** elektrischer Gleichstrom mit einer Stärke von 0,5 mA bis 2 mA verabreicht wird.

9. Verfahren nach einem der vorhergehenden Ansprüche, **dadurch gekennzeichnet, dass** die Stimulation in zeitlich definierten Intervallen, insbesondere in Abständen von 1 Stunde zwischen aufeinanderfolgenden Stimulationen, durchgeführt wird.

10. Verfahren nach einem der vorhergehenden Ansprüche, **dadurch gekennzeichnet dass** die Stimulation nach einem für die zu behandelnde Person charakteristischen Bestromungsprofil ausgeführt wird.

11. Vorrichtung zur Durchführung des Verfahrens nach einem der vorhergehenden Ansprüche,
aufweisend eine im Kopfbereich einer Person befestigbare Haltevorrichtung, welche eine oder mehrere Elektroden aufweist, **dadurch gekennzeichnet, dass** die Haltevorrichtung ein weiches, elastisches, textiles Stirnband aufweist..

12. Vorrichtung nach Anspruch 11, **dadurch gekennzeichnet, dass** die Elektroden ausschließlich von der dem Kopf der Person zugewandten Seite des Stirnbandes sichtbar sind.

13. Vorrichtung nach Anspruch 11, **dadurch gekennzeichnet, dass** die Haltevorrichtung einen Hartkunststoff aufweist.

14. Elektrokosmetische Verwendung einer Vorrichtung, aufweisend eine im Kopfbereich einer Person befestigbare Haltevorrichtung, welche eine oder mehrere Elektroden aufweist, zur Beeinflussung des visuellen Erscheinungsbildes einer Person.
